# FASCICULE DE BREVET EUROPEEN

(11) **EP 3 429 695 B1**
(45) Date de publication et mention de la délivrance du brevet: **22.02.2023**
(21) Numéro de dépôt: 17715637.9
(22) Date de dépôt: 15.03.2017
(51) Int. Cl.: A61K 8/9794, A61Q 19/02, A61Q 19/08, A61K 8/34, A61K 8/49, A61K 8/368

(54) **EXTRAITS DE COQUE DE NOIX DE COCO, COMPOSITIONS L'INCLUANT ET UTILISATIONS**
KOKOSNUSSSCHALENEXTRAKTE ZUSAMMENSETZUNGEN DAMIT UND VERWENDUNGEN
COCONUT SHELL EXTRACTS, COMPOSITIONS CONTAINING SAME AND USES

(30) Priorité: 15.03.2016 FR 1652155
(43) Date de publication de la demande: 23.01.2019
(73) Titulaire: Lubrizol Advanced Materials, Inc., Cleveland, OH 44141 (US)
(72) Inventeur: VITRAC, Caroline, 33650 Saint Medard D'eyrans (FR); VITRAC, Xavier, 33650 Saint Medard D'eyrans (FR)
(74) Mandataire: Aquinov
(86) Numéro de dépôt international: PCT/EP2017/056110
(87) Numéro de publication internationale: WO 2017/158014

(56) Documents cités:
- US-A1- 2008 118 449
- VENKATARAMAN S ET AL: "Antifungal activity of the alcoholic extract of coconut shell - Cocos nucifera Linn", JOURNAL OF ETHNOPHARMACOLOGY, ELSEVIER IRELAND LTD, IE, vol. 2, no. 3, 1 janvier 1980 (1980-01-01) , pages 291-293, XP026649298, ISSN: 0378-8741, DOI: 10.1016/S0378-8741(80)81007-5 [extrait le 1980-01-01]
- TAIWO ADESOLA AKINYELE ET AL: "In-Vitro Antibacterial Properties of Crude Aqueous and n-Hexane Extracts of the Husk of Cocos nucifera", MOLECULES, vol. 16, no. 12, 3 mars 2011 (2011-03-03), pages 2135-2145, XP055296252, DOI: 10.3390/molecules16032135
- DAVID A AKINPELU ET AL: "Probable mechanisms of biocidal action of Cocos nucifera Husk extract and fractions on bacteria isolates", BMC COMPLEMENTARY AND ALTERNATIVE MEDICINE, BIOMED CENTRAL LTD., LONDON, GB, vol. 15, no. 1, 14 avril 2015 (2015-04-14) , page 116, XP021217438, ISSN: 1472-6882, DOI: 10.1186/S12906-015-0634-3
- RODRIGUES ET AL: "Optimization of ultrasound extraction of phenolic compounds from coconut (Cocos nucifera) shell powder by response surface methodology", ULTRASONICS: SONOCHEMISTRY, BUTTERWORTH-HEINEMANN, GB, vol. 15, no. 1, 7 octobre 2007 (2007-10-07), pages 95-100, XP022289498, ISSN: 1350-4177, DOI: 10.1016/J.ULTSONCH.2007.01.006
- GARGI DEY ET AL: "Detection of major phenolic acids from dried mesocarpic husk of mature coconut by thin layer chromatography", INDUSTRIAL CROPS AND PRODUCTS., vol. 18, no. 2, 1 septembre 2003 (2003-09-01), pages 171-176, XP055296379, NL ISSN: 0926-6690, DOI: 10.1016/S0926-6690(03)00056-6
- XIAOXIA LIANG ET AL: "Application of Sub-Critical Water Extraction in Pharmaceutical Industry", JOURNAL OF MATERIALS SCIENCE AND CHEMICAL ENGINEERING, vol. 01, no. 05, 1 janvier 2013 (2013-01-01), pages 1-6, XP055296415, ISSN: 2327-6045, DOI: 10.4236/msce.2013.15001
- TAKAKO YOKOZAWAA AND YOU JUNG KIM: "Piceatannol Inhibits Melanogenesis by Its Antioxidative Actions", BIOLOGICAL & PHARMACEUTICAL BULLETIN (OF JAPAN), PHARMACEUTICAL SOCIETY OF JAPAN, TOKYO, JP, vol. 30, no. 11, 1 novembre 2007 (2007-11-01), pages 2007-2011, XP008165657, ISSN: 0918-6158
- DATABASE WPI Week 201355 8 août 2013 (2013-08-08) Thomson Scientific, London, GB; AN 2013-M13330 XP002760929, & JP 2013 151458 A (MORINAGA & CO LTD) 8 août 2013 (2013-08-08)

## Description

La présente invention concerne l'utilisation dans des compositions à application topique d'un extrait de coque de noix de coco. L'invention a en particulier pour objet un extrait de coque de noix de coco obtenu par extraction solide-liquide avec de l'eau à l'état subcritique, ainsi que des compositions l'incluant et un procédé cosmétique pour améliorer l'état de la peau. La peau, organe le plus étendu du corps humain, forme une barrière de protection de l'organisme contre le milieu extérieur et assure également d'autres fonctions vitales. La recherche cosmétique consiste à proposer des solutions capables de préserver ces fonctions naturelles et à protéger la peau des agressions auxquelles elle est soumise au quotidien. Depuis plusieurs années, sont proposés en particulier des produits d'origine naturelle, notamment issus de matières premières végétales. Néanmoins, ces produits ne sont pas toujours satisfaisants et il subsiste toujours un besoin pour de nouveaux produits naturels capables de protéger et améliorer efficacement l'état de la peau, en particulier des produits capables de lutter contre les signes du vieillissement cutané et des produits dépigmentant et éclaircissant.

L'objectif de la présente invention est de répondre à ce besoin en proposant un extrait naturel de coque de noix de coco.

Le Cocotier (*Cocos nucifera L.*) est une espèce de palmiers de la famille des Arecaceae, présente dans toute la zone intertropicale humide. Il s'agit de la seule espèce du genre Cocos. Le cocotier a une couronne foliaire composée d'une trentaine de palmes vertes. Chaque palme mesure 4 à 7 mètres de long et porte environ 200 folioles de part et d'autre du rachis. Les inflorescences sont composées d'épillets portant des fleurs femelles à leur base et des fleurs mâles à leur extrémité. Les fruits, situés à l'aisselle de chaque palme, sont appelés drupes, ou plus communément « noix de coco ».

La noix de coco est formée de plusieurs enveloppes successives. La plus externe, appelée mésocarpe, bourre, ou coir, est d'abord verte puis brunâtre à maturité. Elle est coriace et fibreuse. Elle sert par exemple à la fabrication de toiles, brosses, paillassons, tapis, matelas, panneaux d'isolation et emballages. Une fois débourrée, la noix présente une coque dure, brune : la graine. De forme oblongue ou sphérique, elle se renforce de trois côtes longitudinales plus ou moins marquées. Une fine pellicule d'un brun rougeâtre, le tégument séminal, forme un lien entre la coque et un albumen blanc ou amande, brillant, de 10 à 15 mm d'épaisseur. Cette sorte de pulpe sert à la préparation du lait de coco, obtenu après broyage de la chair avec de l'eau puis filtration. Cette chair extraite est séchée au soleil jusqu'à la disparition quasi totale de sa teneur en eau qui ne doit pas dépasser 6 %, pour obtenir le coprah. Le coprah sert à la fabrication d'huile de coco, utilisée d'une part dans l'alimentation humaine, pour la confection de margarine, et d'autre part dans la fabrication de savon et de cosmétiques, en particulier du monoï en association avec la fleur de tiare de Tahiti. Plus de 90% de l'huile de coprah se compose de graisses saturées avec des traces de quelques acides gras insaturés. Enfin, un liquide opalescent et sucré occupe jusqu'à trois quarts de la cavité interne de la noix. On l'appelle communément "eau de coco". Ce liquide est la réserve d'eau douce de la noix, destinée à lui permettre de germer quelles que soient les conditions extérieures.

La coque de noix de coco (endocarpe), avec un PCI (pouvoir calorifique inférieur) élevé, une densité importante et une excellente homogénéité est particulièrement intéressante et donc fréquemment utilisée comme combustible, pour l'alimentation de chaudières à déchets par exemple.

En dehors de ces utilisations, la poudre de coques est utilisée dans l'industrie comme charge de matières plastiques ou pour l'élaboration de colles. La carbonisation permet de produire un charbon de coque qui peut être utilisé comme combustible par les particuliers ou servir de matière première pour l'activation et la fabrication de charbon actif. Les propriétés exceptionnelles du charbon actif de coques de noix de coco le destinent à des utilisations comme filtre à eau, à fumées, à gaz, notamment dans les industries de distribution de l'eau, agroalimentaire, nucléaire, etc.

De façon surprenante, selon l'invention, les extraits de coque de noix de coco présentent des effets positifs sur la peau pour la protéger et améliorer ses fonctions naturelles.

L'invention vise donc des compositions cosmétiques et/ou dermocosmétiques destinées à une application topique, comprenant un extrait de coque de noix de coco à titre de principe actif. Appliquées sur la peau, ces compositions permettent d'améliorer son état. Elles permettent notamment de prévenir et/ou réduire et/ou traiter des signes du vieillissement de la peau et/ou des muqueuses et/ou des cheveux, et également d'éclaircir le teint de la peau et/ou de prévenir et/ou réduire les taches brunes de la peau.

Il est déjà connu de l'art antérieur des extraits de coque de noix de coco, tels que des extraits alcooliques décrit par Venkataraman S et al. 1980, mais également des extraits obtenus à partir de l'enveloppe de noix de coco, à froid avec un mélange hydroalcoolique (David A Akinpelu et al. 2015).

Enfin, des compositions cosmétiques contenant du picéatannol sont également connues de US 2008/118449, toutefois, celles-ci contiennent uniquement du picéatannol.

Or, l'invention a pour objet un extrait de coque de noix de coco particulier comprenant entre 50 et 200 ppm de trans-picéatannol en poids d'extrait liquide lorsque l'extrait est liquide, et entre 0.2 et 0.8% de trans-picéatannol en poids d'extrait sec lorsque l'extrait est sous forme solide, ledit extrait est obtenu par extraction solide-liquide avec de l'eau à l'état subcritique, comme solvant d'extraction. Un procédé d'extraction à l'eau subcritique dans l'industrie pharmaceutique est par exemple décrit par Xiaoxia Liang et al. 2013.

L'invention porte aussi sur un procédé d'obtention spécifique particulièrement adapté pour obtenir un tel extrait. La présence de ces composés phénoliques spécifiques, qui ne sont présents que dans l'endocarpe du fruit (coque), joue un rôle important dans l'effet sur la peau des extraits de coque de noix de coco.

L'invention est maintenant décrite en détail en regard des figures annexées sur lesquelles :
- La Figure 1 représente le chromatogramme à 306 nm de l'extrait de l'exemple 1;
- La Figure 2 représente le chromatogramme à 306 nm de l'extrait de l'exemple 2 selon l'invention ;
- La Figure 3A représente les chromatogrammes à 280 nm de trois extraits (éthanol 96%, eau et méthanol/eau 50/50 (v/v) de haut en bas) de mésocarpe fibreux de noix de coco ;
- La Figure 3B représente les chromatogrammes à 280 nm de trois extraits (éthanol 96%, eau et méthanol/eau 50/50 (v/v) de haut en bas) de coque de noix de coco ;
- La Figure 3C représente les chromatogrammes à 280 nm de trois extraits (éthanol 96%, eau et méthanol/eau 50/50 (v/v) de haut en bas) de pulpe de coco ;
- La Figure 4 représente la courbe dose-réponse de l'inhibition de la glycation par l'extrait de l'exemple 2 ;
- La Figure 5A représente la courbe dose-réponse de l'inhibition de l'activité tyrosinase par l'extrait de l'exemple 1 ;
- La Figure 5B représente la courbe dose-réponse de l'inhibition de l'activité tyrosinase par l'extrait de l'exemple 2.

Dans la présente demande, la forme singulière d'un mot comprend le pluriel, et inversement, à moins que le contexte indique clairement le contraire. Ainsi, les références " un", "une", et "le", "la" comprennent les pluriels de ces termes. Par exemple, la référence à "un extrait" ou "une composition" comprend une pluralité de ces "extraits" ou "compositions".

Par principe actif au sens de l'invention on entend une substance présentant un effet sur la peau permettant d'améliorer son aspect et/ou ses fonctions et/ou de la protéger. Il peut s'agir d'un principe actif cosmétique ou d'un principe actif dermatologique.

Par extrait de coque de noix de coco au sens de l'invention on entend un ensemble de molécules obtenues à partir de coque de noix de coco.

Par coque de noix de coco, on entend l'endocarpe du fruit du cocotier.

Par ppm on entend parties par million ou mg/Kg.

Selon un premier aspect, l'invention a donc pour objet des compositions cosmétiques et/ou dermatologiques à application par voie topique, comprenant à titre de principe actif un extrait de coque de noix de coco obtenu par extraction solide-liquide avec de l'eau à l'état subcritique comme solvant d'extraction.

La composition selon l'invention comprend préférentiellement de 0.01 à 10 % en poids d'extrait de coque de noix de coco par rapport au poids total de la composition.

En plus de l'extrait de coque de noix de coco, la composition selon l'invention comprend également un ou plusieurs excipients cosmétiquement ou dermatologiquement acceptables. Il peut s'agir, à titre d'exemples, de colorants, d'actifs filmogènes, de tensioactifs, de parfums, de conservateurs, d'émulsionnants, de gélifiants, d'huiles végétales, de glycols, de vitamines, d'antioxydants, de filtres UV, etc.

La composition peut éventuellement contenir également un ou plusieurs autres principes actifs cosmétiques et/ou dermatologiques, naturels et/ou synthétiques et d'origine animale et/ou végétale.

La composition peut être obtenue par simple mélange des constituants, ou par tout procédé adapté connu de l'homme du métier.

La composition peut se présenter sous toute forme adaptée à une application topique sur la peau et/ou les muqueuses et/ou les cheveux, en particulier sous forme de crème, d'émulsion, de lait, de pommade, de lotion, d'huile, de solution aqueuse, de solution hydro-alcoolique, de solution glycolique, de poudre, de patch ou de spray.

De façon préférée, l'extrait de coque de noix de coco présent dans les compositions selon l'invention est un extrait contenant des polyphénols, en particulier un extrait comprenant entre 5 et 40 % de polyphénols en poids de matière sèches de l'extrait, dosés selon la méthode de Folin-Ciocalteu.

L'extrait de coque de noix de coco présent dans les compositions selon l'invention peut être obtenu partout procédé d'extraction connu de l'homme du métier adapté aux coques de noix de coco. Il peut se présenter sous forme solide ou liquide. Préférentiellement, il s'agit d'un extrait liquide. Dans ce cas, il est préférentiellement stabilisé avec de la glycérine avant incorporation dans la composition.

Selon un mode de réalisation particulièrement adapté, l'extrait de coque de noix de coco est un extrait particulier, tel que décrit en suivant.

En effet, l'invention vise un extrait de coque de noix de coco particulier, liquide ou solide, comprenant du trans-picéatannol en quantité efficace, en particulier un extrait de coque de noix de coco comprenant au moins entre 50 et 200 ppm de trans-picéatannol en poids d'extrait liquide lorsque l'extrait est sous forme liquide, ou entre 0.2% et 0.8% de *trans-*picéatannol en poids d'extrait sec lorsque l'extrait est sous forme solide (en particulier sous forme de poudre). Le dosage de trans-picéatannol est réalisé par chromatographie liquide haute performance (HPLC) que l'extrait soit sous forme liquide ou solide.

Le picéatannol est un composé dont la structure chimique est similaire à celle du resvératrol, composé dont les effets bénéfiques sur la santé sont déjà connus depuis plusieurs années. La structure chimique du trans-picéatannol est la suivante :

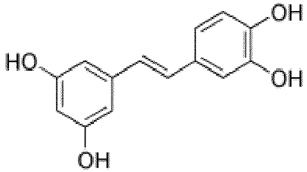

L'extrait de coque de noix de coco comprend au moins une autre molécule en plus du *trans-*picéatannol. Préférentiellement, cette autre molécule (ou ces autres molécules) est (sont) choisie(s) parmi :
- la scirpusine A, de formule : 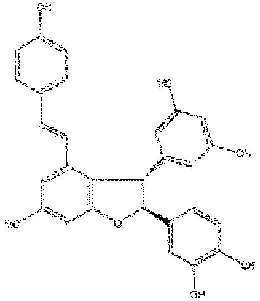
- et/ou la scipusine B, de formule : 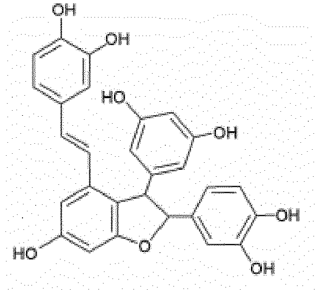
- et/ou l'acide hydroxybenzoïque, en particulier l'acide 4-hydroxybenzoïque de formule :

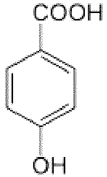

Encore plus préférentiellement, l'extrait de coque de noix de coco comprend :
- entre 2 et 20 ppm de scirpusine A et entre 5 et 50 ppm de scipusine B en poids d'extrait liquide si l'extrait est liquide, soit entre 0.008 et 0.08 % de scirpusine A et entre 0.02 et 0.2% de scipusine B en poids d'extrait sec si l'extrait est solide, et/ou
- entre 10 et 1000 ppm d'acide hydroxybenzoïque en poids d'extrait liquide, soit entre 0.04 % et 4 % d'acide hydroxybenzoïque en poids d'extrait sec si l'extrait est solide

L'extrait de coque de noix de coco selon l'invention comprend préférentiellement entre 5 et 40 % de polyphénols en poids de matière sèches de l'extrait quelle que soit la forme de l'extrait.

L'extrait de coque de noix de coco selon l'invention, pour pouvoir contenir entre 50 et 200 ppm de trans-picéatannol en poids d'extrait liquide, est obtenu par extraction solide-liquide avec de l'eau à l'état subcritique comme solvant d'extraction. De même, la coque de noix de coco est préférentiellement séchée et/ou broyée avant d'être extraite.

L'extrait de coque de noix de coco selon l'invention est ainsi obtenu par extraction à l'eau subcritique. Sous certaines conditions de température et de pression, les propriétés physico-chimiques de l'eau sont modifiées et l'eau passe à l'état subcritique. A des températures supérieures à 100°C mais inférieures au point critique (374°C) et à des pressions inférieures à 221 bars, l'eau reste à l'état liquide mais se transforme en un solvant aux propriétés particulièrement intéressantes dans le domaine de l'extraction végétale. Dans ces conditions, la viscosité et la tension de surface de l'eau subcritique sont inférieures à celle de l'eau à température ambiante, sa diffusivité augmente et sa constante diélectrique devient proche de celle de solvants organiques tels que l'éthanol ou l'acétone. Il en résulte une meilleure pénétration du solvant dans la matrice végétale, un transfert de masse amélioré et un taux de diffusion plus rapide donc des rendements d'extraction plus élevés avec des temps de contact très courts.

Dans le cas d'une extraction à l'eau subcritique, la coque de noix de coco est également préférentiellement séchée et/ou broyée avant d'être extraite.

De façon préférée, l'extrait de coque de noix de coco selon l'invention est obtenu par un procédé d'obtention qui consiste en une extraction à l'eau à l'état subcritique en mode dynamique ou en batch, réalisée à une température comprise entre 100°C et 220°C et à une pression supérieure à 20 bars, suffisante pour maintenir l'eau à l'état liquide. En particulier, l'extraction en mode dynamique est réalisée par la mise en oeuvre des étapes suivantes :
- préférentiellement broyage de la matière végétale, par exemple à l'aide d'un concasseur, afin d'obtenir des particules d'une granulométrie de 0.5 à 10 mm,
- entre 15 et 20 g de coque de noix de coco broyée est introduite dans un réacteur de volume 30 mL, lui-même placé dans une étuve préalablement chauffée à une température supérieure à 100°C, puis l'eau est pompée au travers du réacteur, dans les conditions opératoires suivantes:
- débit: entre 1 et 10 ml/min, préférentiellement entre 2 et 6 ml/min,
- température de l'eau: entre 100 et 220°C, préférentiellement entre 120 et 200°C,
- pression: entre 20 et 50 bars, préférentiellement 30 bars.
- après passage de 100 à 150 mL d'eau à l'état subcritique à travers la matière végétale, l'extrait aqueux est récupéré.

Un des avantages de ce procédé est qu'aucune étape de séparation des parties solides et du liquide n'est nécessaire.

Dans le cas d'une formulation liquide, l'extrait aqueux est mélangé à de la glycérine dans des proportions comprises entre 50 et 80 % en poids de glycérine, afin d'être stabilisé microbiologiquement.

Dans le cas d'une formulation sous forme de poudre, l'extrait aqueux est directement séché par lyophilisation ou atomisation ou dans une étuve sous vide.

Les extraits de coque de noix de coco et en particulier les extraits de coque de noix de coco selon l'invention présentent des propriétés lui permettant d'améliorer l'état de la peau et de maintenir ou rétablir ses fonctions naturelles et de la protéger des agressions extérieures, toutes ces applications chez des personnes saines non malades.

Les extraits de coque de noix de coco et en particulier les extraits de coque de noix de coco selon l'invention présentent notamment :
- un effet antioxydant,
- un effet anti-glycation,
- un effet inhibiteur de la tyrosinase, et
- un effet réparateur de l'ADN des cellules.

Les compositions et les extraits selon l'invention peuvent donc être utilisés dans des procédés cosmétiques, et l'invention vise spécifiquement un procédé cosmétique pour le soin de la peau qui consiste à appliquer sur la peau (en application topique) une composition cosmétique comprenant un extrait de coque de noix de coco.

En particulier, l'invention peut être utilisée pour la mise en oeuvre de procédé cosmétique pour prévenir et/ou réduire et/ou traiter des signes du vieillissement de la peau et/ou des muqueuses et/ou des cheveux.

Différents mécanismes interviennent au cours du vieillissement cutané : les processus oxydatifs, la glycation des protéines, la protéolyse non contrôlée des macromolécules de la matrice extracellulaire, les erreurs commises lors des mécanismes de réparation de l'ADN, etc...

Un des mécanismes impliqué dans le vieillissement correspond à la théorie radicalaire selon laquelle des molécules très réactives, liées à l'oxygène, sont responsables d'effets cellulaires délétères. Ces molécules très réactives sont appelées radicaux libres ou Espèces Oxygénées Réactives (EOR) ou ROS en anglais pour Reactive Oxygen Species. Les radicaux libres sont des molécules instables produites par les mitochondries lors de réactions enzymatiques. Ils sont générés par le fonctionnement normal de l'organisme. Certains autres facteurs entraînent l'augmentation de la production de radicaux libres (tabac, pollution, soleil, pesticides, stress, alimentation déséquilibrée riche en sucres et graisses...). Ces espèces chimiques ont une grande réactivité et peuvent provoquer d'importants désordres moléculaires : péroxydation lipidique des membranes biologiques, altérations protéiques portant à la fois sur les protéines de structure et les protéines plus fonctionnelles comme les enzymes ou les hormones. Ainsi, l'action des radicaux libres contribue à accélérer le processus de vieillissement.

Les protéines peuvent être altérées par des radicaux libres mais également par glycation. La glycation est une réaction non enzymatique entre un sucre réducteur (glucose ou fructose) et le groupement amine libre d'un acide aminé, généralement de la lysine ou de l'arginine. Procédant en plusieurs étapes, la glycation génère, dans ses phases précoces, la formation d'une liaison aldimine instable (base de Schiff), capable de subir un réarrangement moléculaire appelé réarrangement d'Amadori, conduisant à la formation d'une liaison cétoamine stable. Les produits formés sont appelés produits d'Amadori (dans le cas des aldoses), ou de Heyns (dans le cas plus rare des cétoses). Ultérieurement, ils subissent de nombreuses réactions oxydatives, formant des intermédiaires réactifs (notamment des aldéhydes comme le méthylglyoxal) et aboutissant à la formation de composés de structure complexe, souvent fluorescents, dénommés produits de glycation avancés (AGE, Advanced-Glycation End product). La glycation modifie la structure des protéines (formation de liaisons croisées, modifications d'antigénicité), mais également leurs fonctions (activité enzymatique ou biologique), et leurs rapports avec les cellules, en premier lieu avec les cellules inflammatoires.

Les produits de glycation sont principalement détectés sur les molécules ayant un taux de renouvellement lent, comme les collagène I et III ou l'élastine. Au cours du vieillissement, les liaisons intra et intermoléculaires vont s'accumuler, ce qui va provoquer des modifications des propriétés biomécaniques de la peau avec une altération de l'élasticité associée à une rigidification conduisant à l'apparition des rides.

Les liens entre glycation et stress oxydant sont très étroits, et les deux phénomènes sont souvent regroupés sous le terme de " glycoxydation ". Tous les stades de la glycoxydation génèrent la production de radicaux libres oxygénés, et certaines étapes sont communes avec celles de la peroxydation lipidique.

Ainsi, en agissant à la fois sur le piégeage des radicaux libres et sur l'inhibition de la glycation des protéines, les extraits et les compositions selon l'invention peuvent retarder le vieillissement de la peau.

Par ailleurs, l'extrait et/ou la composition selon l'invention peuvent être utilisés pour la mise en oeuvre de procédé cosmétique pour éclaircir le teint de la peau et/ou pour prévenir et/ou réduire les tâches brunes de la peau. La pigmentation naturelle de la peau est due à la production de mélanines par les mélanocytes de l'épiderme. La synthèse de mélanine (mélanogénèse) est augmentée sous l'action des rayons UV ce qui induit le bronzage dont la fonction physiologique est de protéger la peau contre les UV. Divers dysfonctionnements du mécanisme de production de mélanine (par excès d'agressions extérieures, par perturbations hormonales ou par vieillissement) provoquent l'apparition de tâches brunes, notamment sous formes d'éphélides (tâches de rousseur), de tâches solaires ou de tâches de sénescence (respectivement lentigos solaires et lentigos séniles). Pour agir sur la pigmentation de la peau, on peut utiliser des produits qui altèrent la mélanine ou des produits qui inhibent sa synthèse par les mélanocytes ou sa distribution dans les couches de cellules épidermiques. Dans la chaîne de biosynthèse de la mélanine, diverses enzymes et produits intermédiaires peuvent servir de cibles aux inhibiteurs. C'est le cas en particulier de la tyrosinase qui catalyse la formation de dopaquinone puis de dopachrome à partir de L-DOPA. Ainsi, en agissant sur l'activité de la tyrosinase dans les cellules de la peau, les extraits et les compositions selon l'invention présentent un effet dépigmentant de la peau.

L'invention est à présent illustrée à travers des exemples et résultats d'essais.

### EXEMPLES

### Exemple 1 : Extrait de coque de noix de coco obtenu par extraction solide-liquide avec un solvant hydro-alcoolique, hors invention et présent qu'à titre illustratif.

Ce premier exemple d'extrait hors invention est obtenu par un procédé consistant en la mise en oeuvre des étapes suivantes:
- Broyage de la matière végétale à l'aide d'un concasseur afin d'obtenir des particules d'une granulométrie de 1 à 2 mm ;
- Extraction solide/liquide: on mélange 16.5 g de coque de noix de coco broyée dans 100 ml d'une solution hydro-alcoolique méthanol/eau 50/50 (v/v) à température ambiante pendant 24 heures ;
- Séparation des parties solides et du liquide par centrifugation à 9000 tours/minute pendant 10 minutes ;
- Elimination du solvant organique par concentration sous vide au Rotavapor ;
- Séchage du concentré aqueux dans une étuve sous vide.

L'extrait polyphénolique sous forme de poudre est solubilisé dans de l'eau ultra-pure à raison de 10 mg/mL, puis la solution est filtrée sur filtre PTFE 0.45 µm. 10 µl de solution sont alors injectés sur une colonne Prontosil (C18, phase inverse, 250 x 4.6 mm, 5 µm). Les solvants utilisés sont : A: H₂O/TFA 1% (1 L/5 mL) et B: ACN/TFA 1% (1 L/5 mL). Le gradient de séparation est le suivant, à un débit de 0.8 mL/minute :

**Tableau 1: Gradient de séparation utilisé en HPLC**

| **Temps (min.)** | **%B** |
|---|---|
| 0 | 0 |
| 35 | 55 |
| 36 | 100 |
| 37 | 100 |
| 38 | 0 |
| 40 | 0 |

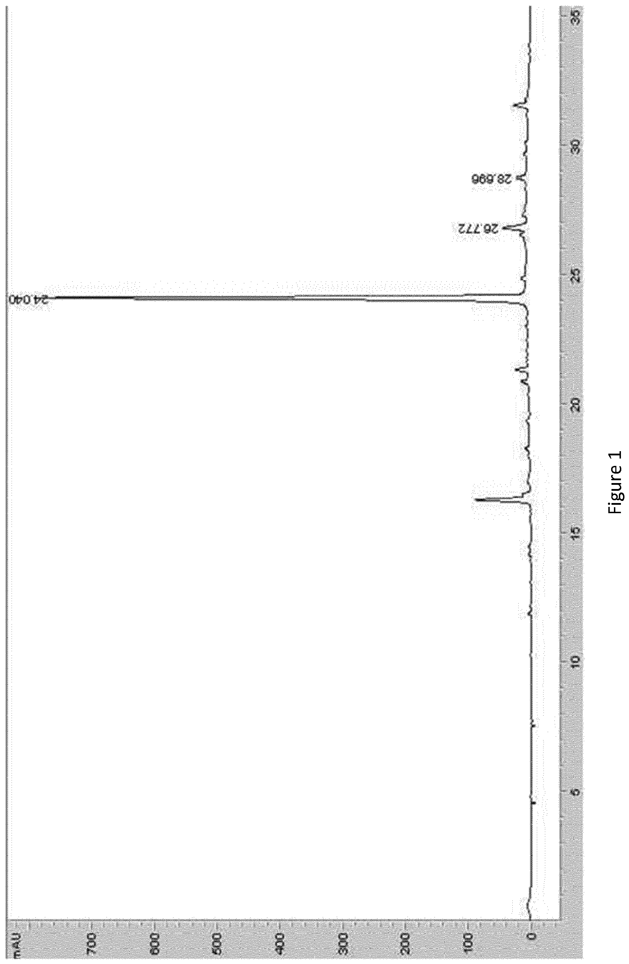

Le chromatogramme à 306 nm de l'extrait obtenu est représenté sur la Figure 1.

L'extrait obtenu présente une teneur en polyphénols totaux de 0.542 mg/mL d'extrait liquide et une teneur en trans-picéatannol de 112.8 ppm (temps de rétention 24'0).

Il présente également une teneur en acide 4-hydroxybenzoïque de 16.8 ppm (temps de rétention 16'8), ainsi qu'une teneur en scirpusine A de 3.7 ppm (temps de rétention 28'6) et une teneur en scirpusine B de 9.0 ppm (temps de rétention 26'7).

### Exemple 2 : Extrait de coque de noix de coco obtenu par extraction à l'eau subcritique, selon l'invention

Le procédé d'extraction de l'exemple 2 est mis en oeuvre de la façon suivante : 16.5 grammes de coques de noix de coco broyées (granulométrie de 1 à 2 mm) sont introduits dans un réacteur de 30 ml dans une étude préalablement chauffée à 150°C, puis l'eau est pompée au travers du réacteur, dans les conditions opératoires suivantes :
- débit : 2 mL/minute
- Température de l'eau : 200°C
- Pression : 30 bars
- Volume final d'extrait: 100 mL

L'extrait polyphénolique obtenu est directement analysé en HPLC. Pour cela, la solution est filtrée sur filtre PTFE 0.45 µm et 10 µl de solution sont alors injectés sur une colonne Prontosil (C18, phase inverse, 250 x 4.6 mm, 5 µm). Les solvants utilisés sont: A: H₂O/TFA 1% (1 L/5 mL) et B: ACN/TFA 1% (1 L/5 mL). Le gradient de séparation est le suivant, à un débit de 0.8 mL/minute :

**Tableau 1: Gradient de séparation utilisé en HPLC-DAD**

| **Temps (min.)** | **%B** |
|---|---|
| 0 | 0 |
| 35 | 55 |
| 36 | 100 |
| 37 | 100 |
| 38 | 0 |
| 40 | 0 |

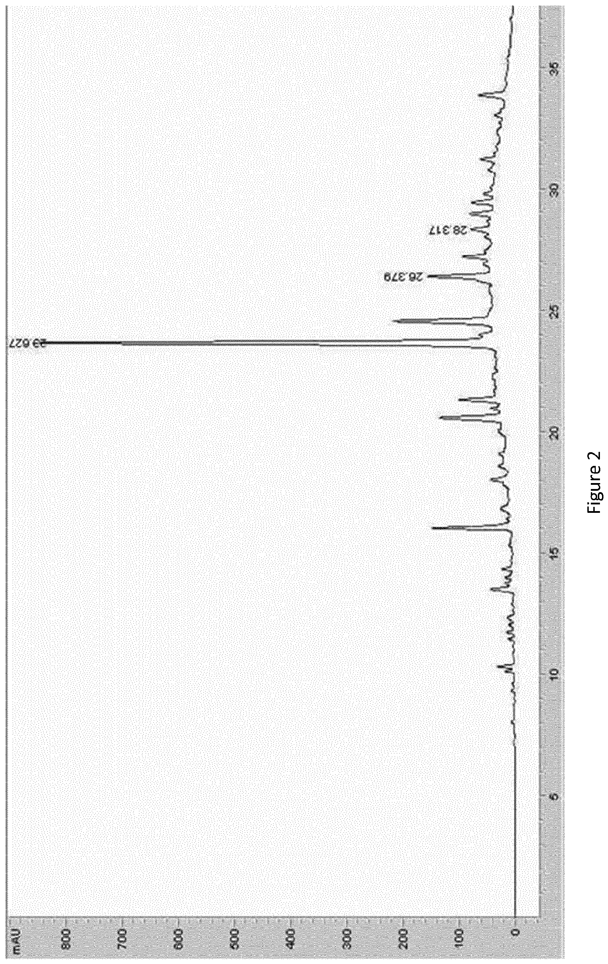

Le profil HPLC (chromatogramme à 306 nm) de l'extrait obtenu est représenté sur la Figure 2. L'extrait obtenu selon ce procédé présente une teneur en polyphénols totaux de 2.43 mg/mL d'extrait liquide et une teneur en trans-picéatannol de 129 ppm (temps de rétention 23'6) L'extrait comprend également 601.5 ppm d'acide 4-hydroxybenzoïque (temps de rétention 17 min), 10.5 ppm de scirpusine A (temps de rétention de 28'3), et 21.3 ppm de scirpusine B (temps de rétention 26'3).

### Exemple 3 : Compositions comprenant un extrait de noix de coco selon l'invention et hors invention (Hl).

Les exemples de compositions données ci-après illustrent l'invention sans en limiter la portée. Les pourcentages sont indiqués en poids par rapport au poids total de la composition.

Une crème protectrice à base d'extrait de coque de coco selon l'exemple 1 (Hors Invention) ayant la composition indiquée ci-après est préparée suivant les techniques usuelles :

### Crème protectrice anti-rides

| **Phase** | **Ingrédient** | **%** |
|---|---|---|
| A | Eau | 80.8 |
| A | Glycérine | 4.5 |
| B | Polymère carboxyvinylique | 0.8 |
| C | Beurre de karité | 1.5 |
| C | Tocophérol | 0.7 |
| C | Caprylic/Capric triglyceride | 7.0 |
| D | Sodium PCA | 1.0 |
| E | Bisabolol | 0.2 |
| F | Extrait de coque de coco (HI) | 3.0 |
| G | Parfum | 0.5 |

La phase aqueuse A est chauffée à 80°C, puis on ajoute la phase B pour former un gel. La phase grasse C est chauffée à 80°C puis mélangée avec le gel sous agitation. Les phases D à F sont ajoutées successivement à 50°C, puis l'ensemble est refroidi progressivement. Enfin, le parfum est ajouté à 40°C.

Cette émulsion peut être utilisée par application bi-quotidienne sur les zones à traiter, en particulier les rides de la patte d'oie et du front.

Un sérum à base d'extrait de coque de coco selon l'exemple 2 ayant la composition indiquée ci-après est préparé suivant les techniques usuelles :

### Sérum éclaircissant

| **Phase** | **Ingrédient** | **%** |
|---|---|---|
| A | Eau | 86.2 |
| A | Glycérine végétale | 3.0 |
| A | Gomme xanthane | 0.5 |
| B | Hyaluronate de sodium | 0.2 |
| B | Squalane végétal | 5.0 |
| B | Parfum | 0.5 |
| B | Alcool cétéarylique | 2.0 |
| C | Extrait de coque de coco (ET) | 2.0 |
| | Glycérine végétale | |
| C | Phenoxyethanol | 0.6 |

Les composants de la phase A sont mélangés à chaud de manière usuelle pour former un gel, puis on ajoute successivement les phases B et C pour former un sérum qui peut être utilisé pour les soins, en particulier pour éclaircir la peau ou traiter les tâches brunes. Ce sérum peut être appliqué une à deux fois par jour sur les zones à traiter.

Une eau de soin à base d'extrait de coque de coco selon l'exemple 2 ayant la composition indiquée ci-après est préparée suivant les techniques usuelles :

### Eau de soin anti-âge

| **Phase** | **Ingrédient** | **%** |
|---|---|---|
| A | Eau | 49.0 |
| A | Hydrolat de Rose centifolia | 40.0 |
| A | Extrait de coque de coco | 10 |
| A | Alcool benzylique (ET) acide déhydroacétique (ET) eau | 0.6 |
| A | Hyaluronate de sodium | 0.4 |

Cette eau de soin peut être appliquée une à deux fois par jour sur les zones à traiter, notamment le visage.

### EVALUATION DES CARACTÉRISTIQUES ET DE L'EFFICACITE DES EXTRAITS ET COMPOSITIONS SELON L'INVENTION Essai 1 : Particularité de la coque de noix de coco : caractérisation des différentes parties de la noix (bourre, coque, pulpe)

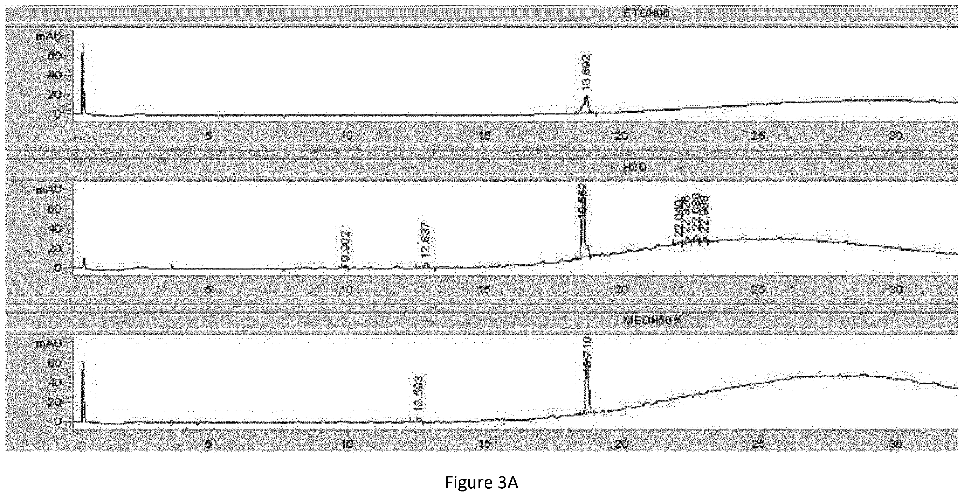

Les différentes parties d'une noix de coco (origine : île de la Réunion) sont séparées afin d'être analysées distinctement : mésocarpe fibreux (bourre), endocarpe (coque) et albumen (pulpe). Environ 1 gramme de chaque partie sont extraits par 5 ml de solvant (Eau ultra pure ou méthanol/eau 50/50 (v/v) ou éthanol 96%) pendant 30 minutes aux ultrasons puis une nuit sous agitation à température ambiante. Après centrifugation (9000 rpm, 10 minutes), le surnageant est filtré (0.45 µm, Millipore) puis 15 µL sont injectés en HPLC avec détection DAD. Les chromatogrammes à 280 nm des différents extraits issus des différentes parties du fruit sont présentés sur les Figures 3A (bourre), 3B (coque) et 3C (pulpe).

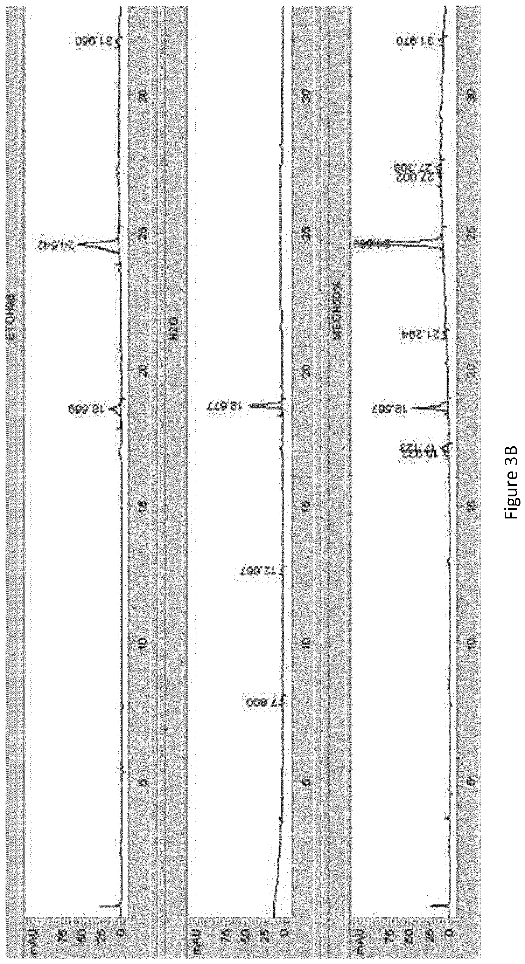

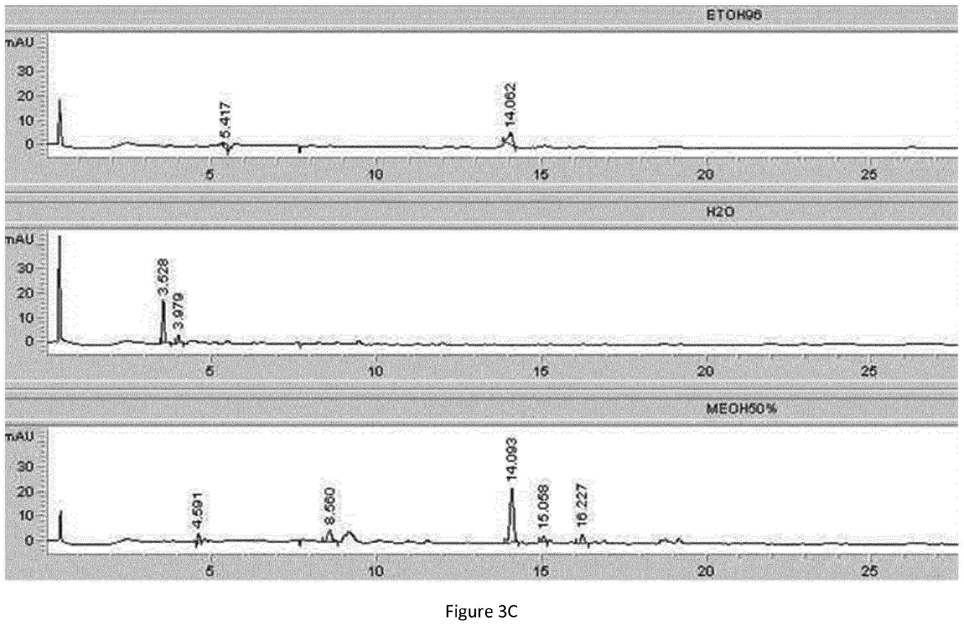

Comme représenté sur la Figure 3B , dans le cas de l'extrait de coque, un pic a été détecté à environ 24'5 minutes. Ce composé a pu être identifié par comparaison avec un standard commercial (AG-CN2-0086-M025, Coger) comme étant du trans-picéatannol. Comme le montrent les Figures 3A et 3C, le trans-picéatannol est absent dans les autres parties du fruit (bourre, pulpe). D'autre part, d'autres composés de la même famille que le picéatannol (stilbènes) ont pu être identifiés dans l'extrait de coque uniquement: il s'agit de deux dimères de picéatannol, la scirpusine A (temps de rétention 29'3) et la scirpusine B (temps de rétention 27'3). Ces composés ont pu être identifiés par comparaison avec des standards commerciaux (LGC standards, ref 57349-84 et ref 57348-94). Enfin, la Figure 3B montre qu'un composé en quantité importante a été détecté au temps de rétention 17'5. Ce composé a pu être identifié, par comparaison avec un standard commercial (Sigma-Aldrich, ref 240141), comme étant l'acide 4-hydroxybenzoïque.

### Essai 2 : Evaluation de l'effet antioxydant des extraits de coque de noix de coco

Les propriétés antioxydantes des extraits de coques de l'exemple 1 (Hors Invention) et de l'exemple 2 ont été évaluées en utilisant les tests chimiques ORAC et DPPH.

Le test ORAC est basé sur la prévention de l'oxydation d'une sonde fluorescente, la fluorescéine, par des radicaux peroxyles générés par un oxydant, l'AAPH (2,2'-azobis (2-methylpropionamidine) dihydrochloride). Ce test est appliqué selon la méthode de Ou (Ou et al., 2001), modifiée par Dàvalos (Dàvalos et al., 2004). La réaction est réalisée en plaque 96 puits dans du tampon phosphate. La fluorescéine est ajoutée à la solution d'extrait à tester et la plaque est pré-incubée 25 minutes à 37°C. La solution d'AAPH est ajoutée et la fluorescence est enregistrée pendant 90 minutes aux longueurs d'onde d'excitation et d'émission de 485 et 530 nm respectivement. Une gamme étalon de Trolox^{®} est réalisée dans les mêmes conditions. L'aire sous courbe (Area Under Curve : AUC) est calculée pour chaque échantillon par intégration de la courbe de fluorescence relative. Une droite de régression est établie entre l'aire sous courbe et la concentration en Trolox^{®} ; le potentiel antioxydant des extraits testés est exprimé en concentration équivalente de Trolox^{®} (µmole / 100 g d'extrait liquide). Concernant le test DPPH: le radical DPPH·+ (2,2-diphenyl-1-picryl-hydrazyl) est stable à température ordinaire et présente une couleur bleue bien caractéristique. Les antioxydants présents dans les extraits à tester le réduisent ce qui entraîne une décoloration facilement mesurable par spectrophotométrie à 517 nm. La méthode est généralement standardisée par rapport au Trolox^{®}. Pour l'évaluation de l'activité antioxydante, la réactivité est estimée par la concentration effective (EC₅₀) de l'antioxydant, qui correspond à une réduction de 50 % de l'activité (de l'absorbance) du DPPH dans le milieu réactionnel. L'EC₅₀ est exprimée en concentration de l'extrait liquide (%).

Les résultats obtenus sont présentés dans le tableau 1 ci-dessous :

**Tableau 1: Activité antioxydantes des extraits de coque de coco**

| **Extrait** | **ORAC (µmole Trolox^{®}/100 g)** | **DPPH (EC₅₀, %)** |
|---|---|---|
| **Exemple 1** | 1215 ± 92 | 1.04 ± 0.06 |
| **Exemple 2** | 9330 ± 432 | 0.23 ± 0.01 |

Ces résultats montrent que les extraits de coque de noix de coco possèdent une activité antioxydante importante. En outre, on constate que l'extrait selon l'invention à l'eau subcritique possède une activité antioxydante nettement plus élevée que l'extrait (Hl) obtenu par macération dans le solvant méthanol/eau 50/50 (v/v).

Ainsi, en agissant sur le piégeage des radicaux libres, l'extrait selon l'invention peut retarder les effets du vieillissement de la peau.

### Essai 3 : Evaluation de l'effet sur l'inhibition de la glycation

Un modèle *in tubo* a été retenu pour évaluer les effets de l'extrait de coque de coco. Il repose sur la mesure de la formation des dérivés de la réaction de glycation entre les groupements aminés libres de l'albumine bovine et le glucose. Certains de ces dérivés, les produits terminaux de glycation avancés (AGE), sont fluorescents. Une molécule de référence, l'aminoguanidine, est testée en parallèle.

Le système d'essai est le mélange réactionnel contenant de l'albumine bovine (1 mg/ml) et du glucose (1.25 M) dans un tampon phosphate D-PBS pH 7.4.

Les produits sont mélangés au système d'essai dans des tubes stériles, puis ils sont placés au bain-marie à 60 °C pendant 24 heures. Après refroidissement, 70 µl d'acide trichloroacétique 100 % (p/v) sont ajoutés au mélange réactionnel puis les tubes sont placés à -20°C pendant 30 minutes. Après centrifugation (10 minutes, 9000 rpm), le culot est repris dans du tampon D-PBS (pH 10). 150 µl de chaque tube sont prélevés et transférés dans une plaque noire 96 puits. La fluorescence est alors lue à l'aide d'un spectrofluorimètre ( FLUOstar, BMG Labtech) aux longueurs d'onde d'excitation 370 nm et d'émission 440 nm.

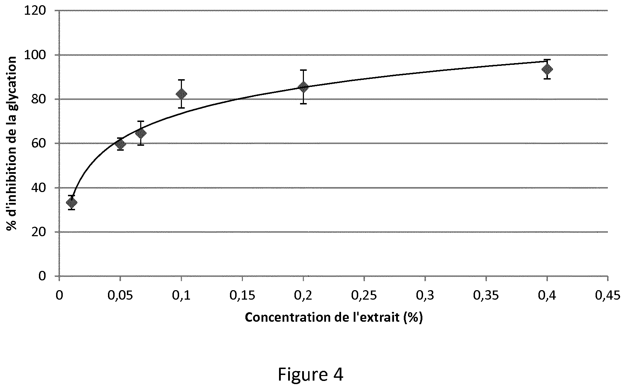

L'extrait de l'exemple 2 a été testé à différentes concentrations : 0.4%, 0.2%, 0.1%, 0.06%, 0.05 % et 0.01%. Les résultats sont présentés sur la Figure 4.

On constate que l'extrait de coque de coco de l'exemple 2, dès la dose 0.05 %, provoque une inhibition de 59.7 % de la glycation de l'albumine induite par le glucose. A plus forte dose (0.4 %), l'extrait de coque de coco de l'exemple 2 provoque une inhibition de 93.5 % de la glycation. Les extraits selon l'invention peuvent donc être utilisés pour des soins anti-âge, notamment pour prévenir le vieillissement de la peau induit par la glycation.

### Essai 4 : Evaluation de l'effet sur l'inhibition de la tyrosinase

Dans la chaîne de biosynthèse de la mélanine, la tyrosinase catalyse la formation de dopaquinone puis de dopachrome à partir de L-DOPA. Le dopachrome est un composé coloré dont on peut suivre l'apparition par spectrophotométrie.

L'utilisation d'inhibiteurs de tyrosinase conduit à limiter la production de mélanine. Cette méthode est couramment utilisée en dermocosmétique pour blanchir et unifier le teint de la peau ou pour estomper les tâches brunes dues au vieillissement cutané. Les molécules décrites comme inhibiteurs de la tyrosinase peuvent agir par plusieurs mécanismes. Elles peuvent interférer avec la transcription de son gène, sa glycosylation, réduire le contrôle post-transcriptionnel, ou inhiber son activité par différentes modalités. La majorité des produits dépigmentants agissent en inhibant l'activité de la tyrosinase. Ces produits peuvent être des analogues de substrats, des inhibiteurs compétitifs ou encore des chélateurs de cuivre, co-facteur indispensable à l'activité tyrosinase.

Dans la présente étude, l'activité anti-tyrosinase des extraits décrits a été mise en évidence sur la tyrosinase de champignon. L'avantage de cette dernière est sa disponibilité dans le commerce sous forme purifiée, ce qui offre la possibilité de conduire des études enzymatiques fiables. Elle est donc utilisée ici comme modèle de screening.

Les extraits de coque de coco à tester sont dilués dans du tampon phosphate de sodium pH 7.2 puis incubé avec la tyrosinase (Tyrosinase from Mushroom > 1000 U/mg (ref Sigma T3824)) à raison de 9 U/puits pendant 5 minutes à 37°C dans une plaque 96 puits. La tyrosine (ref Sigma T2900000, 2 mM) est ensuite ajoutée au mélange et la réaction est suivie par lecture de la DO à 492 nm pendant 5 minutes (apparition du dopachrome) à l'aide d'un spectrophotomètre à lecteur de microplaques. Un témoin négatif est réalisé sans addition d'extrait (tampon).

Le calcul du pourcentage d'inhibition de l'activité tyrosinase par les extraits est effectué en rapportant la DO de l'extrait à la DO du témoin négatif (sans extrait). La dilution des extraits qui inhibent 50% de l'activité tyrosinase (IC₅₀) est calculée à partir de l'équation de la courbe dose-réponse.

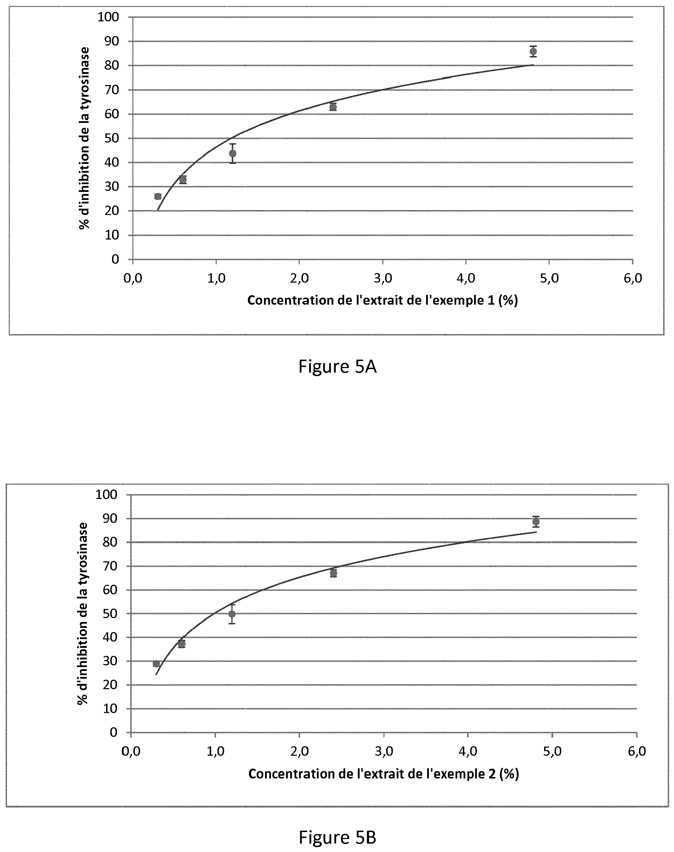

L'extrait de l'exemple 1 (Hors Invention) a été testé à différentes concentrations: 0.3%, 0.6%, 1.2%, 2.4 et 4.8 %. Les résultats sont présentés sur la Figure 5A.

On constate que l'extrait de coque de coco de l'exemple 1, dès la dose 0.6 %, provoque une inhibition de 32.9 % de l'activité tyrosinase. A plus forte dose (1.2 %), l'extrait de coque de coco de l'exemple 1 entraine 50 % d'inhibition de la tyrosinase.

L'extrait de l'exemple 2 a été testé à différentes concentrations: 0.3%, 0.6%, 1.2%, 2.4 et 4.8 %. Les résultats sont présentés sur la Figure 5B.

On constate que l'extrait de coque de coco de l'exemple 2, dès la dose 0.6 %, provoque une inhibition de 37.2 % de l'activité tyrosinase. A plus forte dose (1 %), l'extrait de coque de coco de l'exemple 2 entraine 50 % d'inhibition de la tyrosinase.

L'extrait de coque de coco selon l'invention peut donc être utilisé pour des applications dépigmentantes de la peau.

### Essai 5: Evaluation de l'effet sur l'expression des gènes dans des kératinocytes humains

Les effets de l'extrait de coque de coco selon l'invention (extrait de l'exemple 2) ont été recherchés sur l'expression de gènes dans des kératinocytes épidermiques humains normaux (NHEK). Plus précisément, une analyse transcriptomique a été réalisée en utilisant la plateforme Affymetrix GeneAtlas et la puce « full transcriptome humain » U219 contenant 36000 transcrits et variants.

Les kératinocytes ont été ensemencés en plaque 24 puits (NHEK) et cultivés en milieu de culture pendant 48 heures avec renouvellement du milieu après 24 heures d'incubation, puis en milieu d'essai pendant 24 heures supplémentaires. Le milieu a ensuite été remplacé par du milieu d'essai contenant ou non (Témoin) l'extrait de coque de coco, puis les cellules ont été incubées pendant 24 heures. Toutes les conditions expérimentales ont été réalisées en n=3. A la fin de l'incubation, les surnageants de culture ont été éliminés et les tapis cellulaires ont été rincés avec une solution de PBS. Les plaques ont été immédiatement congelées à sec à - 80°C. Avant l'extraction, les réplicats de culture ont été poolés. L'ARN total de chaque échantillon a été extrait à l'aide de TriPure Isolation Reagent^{®} selon le protocole préconisé par le fournisseur. La quantité et la qualité des ARN ont été évaluées par électrophorèse capillaire (Bioanalyzer 2100, Agilent). La synthèse des ARN anti-sens (ARNa) biotinylés a été réalisée à l'aide du kit « GeneChip 3'IVT Express » (Affymetrix^{®}). Pour chaque échantillon d'ARNa biotinylé un profil électrophorétique a été réalisé (Bioanalyzer 2100, Agilent) avant et après fragmentation. L'hybridation des ARNa marqués et fragmentés sur la puce Affymetrix^{®} U219 chip (36,000 transcripts and variants) a été réalisée sur la station d'hybridation GeneAtlasTM fluidics Affymetrix^{®} hybridization station pendant 20 heures à 45°C. Les puces U219 ont ensuite été scannées à l'aide du GeneAtlasTM Imaging station (Affymetrix^{®} - resolution 2 µm) afin de générer les données d'intensité de signal. Les données d'intensité de signal ont été normalisées à l'aide du logiciel Expression Console (Affymetrix^{®}), à partir de l'algorithme RMA. Un contrôle qualité du marquage ainsi que de l'hybridation a ensuite été réalisé. Les seuils de « fold change » (valeur correspondant au ratio: valeur d'intensité de signal d'une sonde correspondant à l'échantillon traité / valeur d'intensité de signal d'une sonde correspondant au Témoin) ont été définis et appliqués aux données normalisées. Pour une surexpression de la sonde, le seuil de Fold Change a été fixé à >_ 2 et pour une sous-expression de la sonde, le seuil de Fold Change a été fixé à ≤ 0.5.

### Effets sur les kératinocytes épidermiques:

Les kératinocytes humains normaux (NHEK) ont été traités pendant 24 heures par l'extrait de coque de coco de l'exemple 2 à la dose 0.123 % (p/v). A la fin de l'incubation, l'expression génique a été analysée par comparaison avec l'échantillon Témoin (non traité).

Comme le montre le Tableau 2, l'extrait de coque de coco de l'exemple 2 a très fortement augmenté l'expression de certains marqueurs de détoxification cellulaire et de réparation de l'ADN :

**Tableau 2 : Expression relative des gènes dans les kératinocytes traités ou non par l'extrait de coque de coco de l'exemple 2**

| Gène | Témoin (non traité) | Extrait coque de coco (0.123%) | Fold change |
|---|---|---|---|
| **CYP1B1** | 26,72 | 307,04 | 11,49 |
| **CYP1A1** | 52,40 | 484,33 | 9,24 |
| **RAD23B** | 115,53 | 372,82 | 3,23 |

- **CYP1B1 et CYP1A1 (cytochrome P450):** les gènes CYP1B1 et CYP1A1 codent pour des enzymes de la superfamille des cytochromes P450. Les cytochromes P450 sont des hémoprotéines qui interviennent dans des réactions d'oxydo-réduction de nombreuses molécules, qu'il s'agisse de métabolites ou de xénobiotiques (polluants, toxines, drogues, médicaments, etc..).
- **RAD23B:** il s'agit d'un gène codant pour une protéine de réparation par excision de nucléotides ou NER (pour nucleotide excision repair), un des systèmes naturels permettant la réparation de l'ADN dégradé (en particulier par une exposition aux ultraviolets). Il freine le vieillissement de l'organisme, limite les mutations délétères et le risque d'apparition de tumeurs et cancer.

Ces résultats sont en faveur d'un renforcement des mécanismes de défense cellulaire, notamment de la détoxification et la réparation de l'ADN au niveau des cellules de l'épiderme par l'extrait de coque de coco.

## Revendications

1. Extrait de coque de noix de coco liquide ou solide **caractérisé en ce qu'**il comprend du trans-picéatannol en une quantité représentant :
- entre 50 et 200 ppm en poids d'extrait liquide s'il est liquide, ou
- entre 0.2% et 0.8% en poids d'extrait sec s'il est solide
**caractérisé en ce qu'**il est obtenu par extraction solide-liquide avec de l'eau à l'état subcritique comme solvant d'extraction.

2. Extrait de coque de noix de coco selon la revendication 1, **caractérisé en ce qu'**il comprend entre 2 et 20 ppm de scirpusine A et entre 5 et 50 ppm de scirpusine B en poids d'extrait liquide lorsque l'extrait est liquide ou entre 0.008 et 0.08% de scirpusine A et entre 0.02 et 0.2% de scirpusine B en poids d'extrait sec lorsque l'extrait est solide.

3. Extrait de coque de noix de coco selon la revendication 1 ou 2, **caractérisé en ce qu'**il comprend entre 10 et 1000 ppm d'acide hydroxybenzoïque en poids d'extrait liquide lorsque l'extrait est liquide ou entre 0.04 et 4% d'acide hydroxybenzoïque en poids d'extrait sec lorsque l'extrait est sec.

4. Extrait de coque de noix de coco selon l'une des revendications précédentes, **caractérisé en ce que** la coque de noix de coco est séchée et broyée avant d'être extraite.

5. Procédé d'obtention d'un extrait selon l'une des revendications 1 à 3, **caractérisé en ce qu'**il consiste en une extraction à l'eau subcritique, réalisée à une température comprise entre 100°C et 220°C et à une pression supérieure à 20 bars, suffisante pour maintenir l'eau à l'état liquide.

6. Composition cosmétique et/ou dermatologique à application par voie topique, comprenant à titre de principe actif un extrait de coque de noix de coco comprenant entre 5 et 40 % de polyphénols en poids de matière sèches, l'extrait de coque de noix de coco étant un extrait selon l'une des revendications 1 à 4.

7. Composition cosmétique et/ou dermatologique selon la revendication 6, **caractérisée en ce qu'**elle comprend de 0.01 à 10 % en poids d'extrait de coque de noix de coco par rapport au poids total de la composition

8. Composition cosmétique et/ou dermatologique selon l'une des revendications 6 ou 8, **caractérisée en ce qu'**elle se présente sous forme de crème, d'émulsion, de lait, de pommade, de lotion, d'huile, de solution aqueuse, de solution hydro-alcoolique, de solution glycolique, de poudre, de patch ou de spray.

9. Composition cosmétique et/ou dermatologique selon l'une des revendications 6 à 8, **caractérisée en ce que** l'extrait de coque de noix de coco est un extrait liquide stabilisé avec de la glycérine avant incorporation dans la composition.

10. Procédé cosmétique pour améliorer l'état de la peau, **caractérisé en ce qu'**il consiste à appliquer sur la peau une composition cosmétique selon l'une des revendications 6 à 9.

11. Procédé cosmétique selon la revendication 10, pour prévenir et/ou réduire et/ou traiter des signes du vieillissement de la peau et/ou des muqueuses et/ou des cheveux.

12. Procédé cosmétique selon la revendication 11, pour éclaircir le teint de la peau et/ou pour prévenir et/ou réduire les taches brunes de la peau.

## Patentansprüche

1. Flüssiger oder fester Kokosnussschalenextrakt, **dadurch gekennzeichnet, dass** er trans-Piceatannol in einer Menge umfasst, die darstellt:
- zwischen 50 und 200 ppm, bezogen auf das Gewicht des Flüssigextrakts, wenn er flüssig ist, oder
- zwischen 0,2 % und 0,8 %, bezogen auf das Gewicht des Trockenextrakts, wenn er fest ist
**dadurch gekennzeichnet, dass** er durch Fest-Flüssig-Extraktion mit Wasser in unterkritischem Zustand als Extraktionslösungsmittel erhalten wird.

2. Kokosnussschalenextrakt nach Anspruch 1, **dadurch gekennzeichnet, dass** er zwischen 2 und 20 ppm Scirpusin A und zwischen 5 und 50 ppm Scirpusin B, bezogen auf das Gewicht des Flüssigextrakts, wenn der Extrakt flüssig ist, oder zwischen 0,008 und 0,08 % Scirpusin A und zwischen 0,02 und 0,2 % Scirpusin B, bezogen auf das Gewicht des Trockenextrakts, wenn der Extrakt fest ist, umfasst.

3. Kokosnussschalenextrakt nach Anspruch 1 oder 2, **dadurch gekennzeichnet, dass** er zwischen 10 und 1000 ppm Hydroxybenzoesäure, bezogen auf das Gewicht des Flüssigextrakts, wenn der Extrakt flüssig ist, oder zwischen 0,04 und 4 % Hydroxybenzoesäure, bezogen auf das Gewicht des Trockenextrakts, wenn der Extrakt trocken ist, umfasst.

4. Kokosnussschalenextrakt nach einem der vorstehenden Ansprüche, **dadurch gekennzeichnet, dass** die Kokosnussschale getrocknet und zerkleinert wird, bevor sie extrahiert wird.

5. Verfahren zum Erhalten eines Extrakts nach einem der Ansprüche 1 bis 3, **dadurch gekennzeichnet, dass** es aus einer Extraktion mit unterkritischem Wasser besteht, die bei einer Temperatur zwischen 100 °C und 220 °C und bei einem Druck von mehr als 20 bar durchgeführt wird, der zum Halten des Wassers in dem flüssigen Zustand ausreicht.

6. Kosmetische und/oder dermatologische Zusammensetzung zum Auftragen auf topischem Weg, umfassend als Wirkstoff einen Kokosnussschalenextrakt, umfassend zwischen 5 und 40 % Polyphenole, bezogen auf das Gewicht der Trockensubstanz, wobei der Kokosnussschalenextrakt ein Extrakt nach einem der Ansprüche 1 bis 4 ist.

7. Kosmetische und/oder dermatologische Zusammensetzung nach Anspruch 6, **dadurch gekennzeichnet, dass** sie zu 0,01 bis 10 Gew.-% Kokosnussschalenextrakt umfasst, bezogen auf das Gesamtgewicht der Zusammensetzung

8. Kosmetische und/oder dermatologische Zusammensetzung nach einem der Ansprüche 6 oder 8, **dadurch gekennzeichnet, dass** sie in Form einer Creme, einer Emulsion, einer Milch, einer Salbe, einer Lotion, eines Öls, einer wässrigen Lösung, einer wässrig-alkoholischen Lösung, einer Glykollösung, eines Pulvers, eines Pflasters oder eines Sprays vorliegt.

9. Kosmetische und/oder dermatologische Zusammensetzung nach einem der Ansprüche 6 bis 8, **dadurch gekennzeichnet, dass** der Kokosnussschalenextrakt ein Flüssigextrakt ist, der vor einer Einarbeitung in die Zusammensetzung mit Glycerin stabilisiert wurde.

10. Kosmetisches Verfahren zum Verbessern des Hautzustands, **dadurch gekennzeichnet, dass** es darin besteht, eine kosmetische Zusammensetzung nach einem der Ansprüche 6 bis 9 auf die Haut aufzutragen.

11. Kosmetisches Verfahren nach Anspruch 10 zum Verhindern und/oder Reduzieren und/oder Behandeln von Alterungserscheinungen der Haut und/oder der Schleimhäute und/oder der Haare.

12. Kosmetisches Verfahren nach Anspruch 11 zum Aufhellen des Hauttons und/oder zum Verhindern und/oder Reduzieren von braunen Flecken der Haut.

## Claims

1. Liquid or solid coconut shell extract **characterized in that** it comprises *trans*-piceatannol in an amount of:
- between 50 and 200 ppm by weight of liquid extract if it is liquid, or
- between 0.2% and 0.8% by weight of dry extract if it is solid
**characterized in that** the extract is obtained by solid-liquid extraction using subcritical water as an extraction solvent.

2. Coconut shell extract according to claim 1, **characterized in that** it comprises between 2 and 20 ppm of scirpusin A and between 5 and 50 ppm of scirpusin B by weight of liquid extract when the extract is liquid, or comprises between 0.008 and 0.08% of scirpusin A and between 0.02 and 0.2% of scirpusin B by weight of dry extract when the extract is solid.

3. Coconut shell extract according to either claim 1 or claim 2,
**characterized in that** it comprises between 10 and 1000 ppm of hydroxybenzoic acid by weight of liquid extract when the extract is liquid, or comprises between 0.04 and 4% of hydroxybenzoic acid by weight of dry extract when the extract is dry.

4. Coconut shell extract according to any of the preceding claims, **characterized in that** the coconut shell is dried and ground before being extracted.

5. Method for obtaining an extract according to any of claims 1 to 3, **characterized in that** it consists of subcritical water extraction carried out at a temperature of between 100°C and 220°C and at a pressure greater than 20 bar, which is sufficient for maintaining the water in the liquid state.

6. Cosmetic and/or dermatological composition for topical application, comprising as an active ingredient a coconut shell extract comprising between 5 and 40% of polyphenols by weight of dry matter, the coconut shell extract being an extract according to any of claims 1 to 4.

7. Cosmetic and/or dermatological composition according to claim 6, **characterized in that** it comprises from 0.01 to 10% by weight of coconut shell extract based on the total weight of the composition

8. Cosmetic and/or dermatological composition according to either claim 6 or claim 8, **characterized in that** it is in the form of a cream, emulsion, milk, ointment, lotion, oil, aqueous solution, hydroalcoholic solution, glycolic solution, powder, patch or spray.

9. Cosmetic and/or dermatological composition according to any of claims 6 to 8, **characterized in that** the coconut shell extract is a liquid extract stabilized with glycerin prior to being incorporated into the composition.

10. Cosmetic method for improving the condition of the skin,
**characterized in that** it consists in applying a cosmetic composition according to any of claims 6 to 9 to the skin.

11. Cosmetic method according to claim 10, for preventing and/or reducing and/or treating signs of aging of the skin and/or mucous membranes and/or hair.

12. Cosmetic method according to claim 11, for lightening the complexion of the skin and/or for preventing and/or reducing dark spots on the skin.
